# EUROPÄISCHE PATENTSCHRIFT

(11) **EP 0 768 291 B1**
(45) Veröffentlichungstag und Bekanntmachung des Hinweises auf die Patenterteilung: **03.01.2001**
(21) Anmeldenummer: 96115505.8
(22) Anmeldetag: 27.09.1996
(51) Int. Cl.: C07C 29/80, C07C 33/22, C07C 22/04, C07C 43/164

(54) **Verfahren zur Trennung eines Gemisches aus Benzylchlorid, Benzylalkohol, Dibenzylether und wässriger Salzsäure**
Process for the separation of a mixture of benzyl chloride, benzyl alcohol, dibenzyl ether and aqueous hydrochloric acid
Procédé pour la séparation d'un mélange du chlorure de benzyle, de l'alcool de benzyle, de l'éther dibenzylique et de l'acide hydrochlorique aqueux

(30) Priorität: 10.10.1995 DE 19537752
(43) Veröffentlichungstag der Anmeldung: 16.04.1997
(73) Patentinhaber: BAYER AG, 51368 Leverkusen (DE)
(72) Erfinder: Buysch, Hans-Josef, Dr., 47809 Krefeld (DE); Jansen, Ursula, Dr., 47799 Krefeld (DE); Ooms, Pieter, Dr., 47800 Krefeld (DE); Hoffmann, Erhard-Günther, Dr., 40883 Ratingen (DE); Schenke, Bernd-Ulrich, 46236 Bottrop (DE)

(56) Entgegenhaltungen:
- EP-A- 0 064 486
- US-A- 3 557 222
- CHEMICAL ABSTRACTS, Bd. 98, Nr. 13, 28 März 1983, Columbus, Ohio, US; Zusammenfassung Nr. 106890D, J. SCHREIBER, ET AL.: 'Preparation of benzyl alcohol from benzyl chloride' Seite 565; XP002031399 & CHEMCKY PRUMYSL, Bd. 32, Nr. 11, 1982, Seiten 586-588,
- CHEMICAL ABSTRACTS, vol. 102, no. 5, 4.Februar 1985 Columbus, Ohio, US; abstract no. 45606t, Seite 543; XP002031400 & CS 216 042 B (J. SCHREIBER, ET AL.) 1.September 1984

## Beschreibung

Die vorliegende Erfindung betrifft ein Verfahren zur kontinuierlichen destillativen Trennung von Gemischen aus Benzylchlorid, Benzylalkohol, Dibenzylether und wäßriger Salzsäure, wie sie typischerweise bei der Hydrolyse von Benzylchlorid mit Wasser zu Benzylalkohol entstehen.

In der Literatur beschriebene Herstellungsverfahren von Benzylalkohol durch Hydrolyse von Benzylchlorid wenden in der Regel homogene Mischungen aus Benzylchlorid, Wasser und einem wasserlöslichen Lösungsvermittler, wie Alkohol, Aceton, Dioxan oder Essigsäure an (J.Chem.Soc. 1954, 1840; Ibid. 1957, 4747); der Möglichkeit einer weitgehenden Hydrolyse des Benzylchlorids in solchen Verfahren steht die Notwendigkeit einer aufwendigen destillativen Trennung gegenüber. Die Hydrolyse von Benzylchlorid ist auch in Gegenwart von Alkalien bzw. (Erd)Alkalicarbonaten beschrieben worden (J.Am.Chem.Soc. 62 (1940), 2481). Die Gegenwart solcher alkalischer Hydrolysemittel bewirkt eine Beschleunigung der Hydrolyse. Gleichzeitig kann die Reaktion bis zum vollständigen Umsatz gebracht werden. Nachteilig bei der alkalischen Verseifung ist jedoch der Anfall großer Mengen an wäßrigen Ablaugen mit nichtumgesetztem alkalischen Hydrolysemittel und gebildetem Natriumchlorid. Die Entsorgung solcher Ablaugen ist umständlich und kostspielig.

EP 064 486 A1 beschreibt die Hydrolyse von α-chlorierten Toluolverbindungen, insbesondere von Benzylchlorid in Gegenwart der wäßrigen Lösung eines Alkali- oder Erdalkalihydroxids, -hydrogencarbonats oder -carbonats. Es werden große Mengen eines inerten Lösungsmittels zugegeben, die dazu dienen, die Bildung von Dibenzylether, der bei der Hydrolyse von Benzylchlorid als Nebenprodukt anfällt, zu minimieren. Es wird ein Gemisch erhalten, das im wesentlichen aus Benzylalkohol, inertem Lösungsmittel, Dibenzylether und Benzylchlorid besteht, und durch eine kontinuierliche Destillation aufgetrennt werden kann. Auch hier fallen große Mengen an wäßrigen Ablaugen an. Darüber hinaus muß das eingesetzte inerte Lösungsmittel aus dem Produktgemisch abgetrennt werden.

Bei der salzfreien Hydrolyse von Benzylchlorid mit Wasser ohne Basen wird gemäß einem noch nicht veröffentlichten Verfahren nur ein unvollständiger Umsatz von etwa 35-99 % durchgeführt und danach die wäßrige Phase von der organischen getrennt. Letztere besteht je nach Umsatz aus wechselnden Anteilen an Benzylchlorid, Benzylalkohol, Dibenzylether und Resten an wäßriger Salzsäure, die in der organischen Phase löslich sind oder die nicht vollständig abgetrennt wurden.

In den genannten Verfahren tritt als Reaktionsprodukt neben dem gewünschten Benzylalkohol stets auch Dibenzylether auf; seine Bildung erfolgt im basischen Bereich offensichtlich durch Kondensation von bereits gebildetem Benzylalkohol und noch nicht hydrolysiertem Benzylchlorid. Diese Kondensation tritt jedoch nicht nur während der Hydrolysereaktion auf, sondern erfolgt auch während der destillativen Aufarbeitung eines solchen Hydrolysegemisches, sobald der Gehalt an Benzylchlorid oberhalb von 1 % liegt (Chem.Prum. 32 (1982), 586; zitiert nach C.A. 98: 106 890). Um diese während der Destillation auftretende nachträgliche Bildung von Dibenzylether zu unterdrücken, wird eine aufwendige Umsetzung von Resten an Benzylchlorid mit Stickstoffverbindungen, beispielsweise mit Hexamethylentetramin, empfohlen (CS 216 042 B; zitiert nach C.A. 102, 45606t).

Es wurde nun gefunden, daß man die Bildung von Dibenzylether während der Destillation praktisch vermeiden kann, wenn die organische Phase kontinuierlich über eine Seiteneinspeisung in eine Destillationskolonne eingetragen wird, aus der wäßrige Salzsäure und Benzylchlorid als Kopfprodukt und Benzylalkohol und Dibenzylether als Sumpfprodukt kontinuierlich entnommen werden.

Die Erfindung betrifft ein Verfahren zur Trennung eines Gemisches, das Benzylchlorid, Benzylalkohol, Dibenzylether und wäßrige Salzsäure enthält, das dadurch gekennzeichnet ist, daß ein solches Gemisch einer kontinuierlich betriebenen Destillationskolonne mit Abtriebsteil und Verstärkerteil über eine Seiteneinspeisung zugeführt wird, die Destillationskolonne bei einem Druck von 1-950 mbar am Kopf der Kolonne betrieben wird und am Kopf der Destillationskolonne ein Gemisch, das im wesentlichen aus Benzylchlorid und wäßriger Salzsäure besteht, und aus dem Sumpf der Destillationskolonne ein Gemisch, das im wesentlichen aus Benzylalkohol und Dibenzylether besteht, entnommen werden.

Erfindungsgemäß können Gemische mit den genannten Inhaltsstoffen, die aus unterschiedlichsten Quellen stammen, eingesetzt werden. Üblicherweise stammen einzusetzende Gemische jedoch aus der Hydrolyse von Benzylchlorid zur Herstellung von Benzylalkohol. Insbesondere handelt es sich um Hydrolysegemische, die einer Vorabtrennung in eine wäßrige Phase und eine organische Phase unterworfen wurden, wobei die organische Phase erfindungsgemäß eingesetzt wird. Einzusetzende Gemische bestehen im allgemeinen aus 1 - 65 Gew.-% Benzylchlorid, 34 - 98 Gew.-% Benzylalkohol, 0,5 - 12 Gew.-% Dibenzylether und 0,5 - 12 Gew.-% wäßriger Salzsäure, bevorzugt aus 9 - 60 Gew.-% Benzylchlorid, 39 - 90 Gew.-% Benzylalkohol, 0,5 -10 Gew.-% Dibenzylether und 0,5 - 10 Gew.-% wäßriger Salzsäure, besonders bevorzugt aus 24 -55 Gew.-% Benzylchlorid, 44 - 75 Gew.-% Benzylalkohol, 0,5 -8 Gew.-% Dibenzylether und 0,5 - 8 Gew.-% wäßriger Salzsäure. Alle prozentualen Angaben beziehen sich hierbei auf das Gesamtgewicht der genannten vier Bestandteile.

Je nach Umsatz des zu hydrolysierenden Benzylchlorids und nach eingesetzter Wassermenge enthält die im einzusetzenden Gemisch vorliegende wäßrige Salzsäure 0,01 - 23 Gew.-% an Chlorwasserstoff.

Das einzusetzende und erfindungsgemäß aufzutrennende Gemisch wird einer kontinuierlich betriebenen Destillationskolonne über eine Seiteneinspeisung zugeführt. Die Destillationskolonne wird bei einem Druck von 1 - 950 mbar, bevorzugt 10 - 500 mbar, besonders bevorzugt 20 - 300 mbar am Kopf der Kolonne betrieben. Hierbei stellt sich in einer dem Fachmann bekannten Weise in Abhängigkeit vom eingestellten Kopfdruck und in Abhängigkeit von der Zusammensetzung des aufzutrennenden Gemisches eine Temperatur im Sumpf von 30 - 200°C, bevorzugt 60 - 180°C, besonders bevorzugt 70 - 165°C, ein. In entsprechender Weise stellt sich eine Kopftemperatur von 20 - 175°C, bevorzugt 50 - 155°C, besonders bevorzugt 60 - 140°C, ein. Die Kopftemperatur liegt hierbei stets unter der Sumpftemperatur. Die erfindungsgemäß vorzunehmende Seiteneinspeisung des aufzutrennenden Gemisches wird an einem Punkt der Destillationskolonne vorgenommen, an welchem eine Temperatur von 25 - 195°C, bevorzugt 55 - 175°C, besonders bevorzugt 65 - 160°C, herrscht. Die Einstellung eines Temperaturgradienten ist dem Fachmann bekannt. Er ist u.a. abhängig von der Zusammensetzung des Gemisches sowie dessen Vorerwärmung. Im Rahmen des erfindungsgemäßen Verfahrens kann die Destillationskolonne mit einer Belastung von 0,05 - 1,0 kg organische Phase im aufzutrennenden Gemisch pro Liter Leervolumen der Kolonne pro Stunde betrieben werden. Bevorzugt wird eine Belastung von 0,15 - 0,9 kg/l•h, besonders bevorzugt 0,25 - 0,8 kg/l•h, eingestellt.

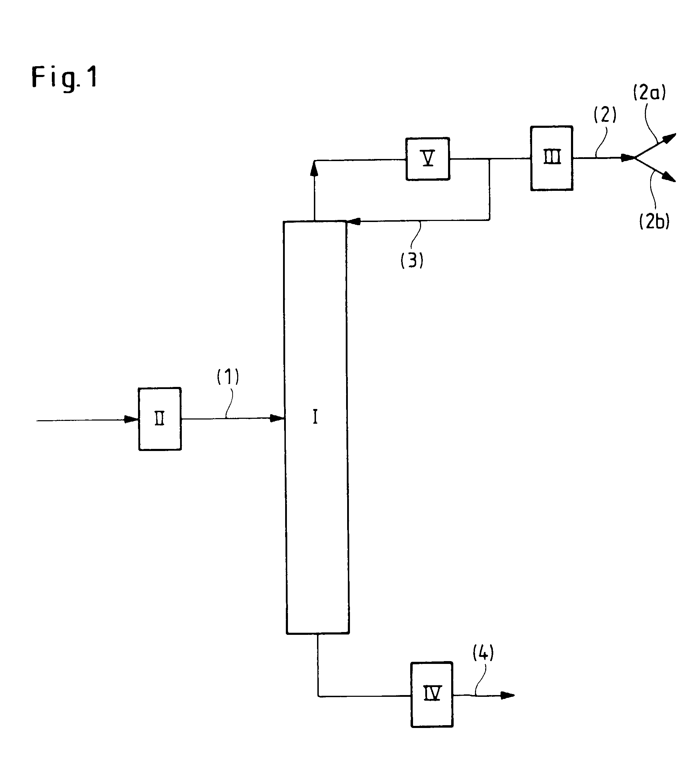

Die zusätzliche Bildung von Dibenzylether wird erfindungsgemäß praktisch verhindert und auf geringe Werte von weniger als 4 %, bevorzugt weniger als 2 %, besonders bevorzugt weniger als 0,5 %, bezogen auf die Menge an Benzylalkohol, gesenkt. Fig. 1 zeigt ein Prinzipschema für die Durchführung des erfindungsgemäßen Verfahrens. Sie enthält als Apparate: Eine Destillationskolonne (I), eine Speisepumpe für das aufzutrennende Gemische (II), 2 Pumpen (III + IV) zur Entnahme des Kopf- bzw. Sumpfproduktes, einen Kondensator für das Kopfprodukt (V). Fig. 1 zeigt als Stoffströme: das aufzutrennende Ausgangsgemisch (1), das Kopfprodukt (2), bestehend aus wäßriger Salzsäure (2a) und Benzylchlorid (2b), eine Rückflußleitung (3) auf die Kolonne (I), und das Sumpfprodukt (4), bestehend aus Benzylalkohol und Dibenzylether. (I) kann eine Destillationskolonne mit bekannten Einbauten wie Glockenböden, Siebböden usw., oder eine mit Füllkörpern gepackte Destillationskolonne sein. Solche Kolonnen sind dem Fachmann ebenfalls bekannt.

Das Sumpfprodukt kann in einer dem Fachmann bekannten Weise, beispielsweise durch Destillation, Kristallisation oder Ausfrieren in den gewünschten Benzylalkohol und das Nebenprodukt Dibenzylether getrennt werden. Das Kopfprodukt kann in einer ebenfalls dem Fachmann bekannten Weise, beispielsweise durch Dekantieren in Benzylchlorid und wäßrige Salzsäure getrennt werden. Das Benzylchlorid kann in die Hydrolyse zurückgeführt werden. Die wäßrige Salzsäure kann ebenfalls einer fachmännisch bekannten Verwendung zugeführt werden.

Gegenüber dem Stand der Technik bietet das erfindungsgemäße Verfahren eine Reihe von Vorteilen:
a) Reste an Benzylchlorid im Hydrolysegemisch müssen nicht umständlich durch chemische Umsetzungen entfernt werden;
b) durch die kontinuierliche Seiteneinspeisung und Vakuumdestillation eines Hydrolysegemisches gelingt es, die zusätzliche Bildung von Dibenzylether in der oben angegebenen Weise praktisch vollständig zu unterdrücken;
c) simultan wird eine praktisch vollständige Abtrennung des Benzylchlorids vom Benzylalkohol erreicht, so daß der Gehalt an Benzylchlorid unterhalb von 50 ppm liegt.

### Beispiele

### Beispiel 1

Als Reaktionsapparat wurde eine Destillationskolonne mit Seiteneinspeisung, die aus einem Abtriebsteil und einem Verstärkerteil bestand, eingesetzt. Zum Anfahren der Kolonne wurde der Sumpf mit reinem Benzylalkohol beschickt und bei 100 mbar auf 142°C erhitzt. Sobald an der Einspeisestelle eine Temperatur von 135°C erreicht worden war, wurde mit der Dosierung eines Gemisches aus 35 Gew.-% Benzylchlorid, 57 Gew.-% Benzylalkohol, 4 Gew.-% Dibenzylether und 4 Gew.-% 3 %iger Salzsäure begonnen. Die Belastung betrug 0,145 kg/l•h. Benzylchlorid und verdünnte Salzsäure destillierten bei einer Temperatur von 104°C über Kopf ab, während Benzylalkohol und Dibenzylether in den Sumpf gingen, von wo aus sie kontinuierlich ausgefördert wurden. Nach der Trennung des kondensierten Kopfproduktes in wäßrige Salzsäure und organische Phase lag die Zusammensetzung der organischen Phase nach Einstellung des Destillationsgleichgewichts bei mehr als 95 Gew.-% Benzylchlorid und weniger als 5 Gew.-% Benzylalkohol. Die Sumpfphase enthielt neben Benzylalkohol nur Dibenzylether in einer Menge von ca. 8,0 Gew.-%, was einem Zuwachs an Dibenzylether von 1,4 Gew.-%, bezogen auf Benzylalkohol, entsprach. Benzylchlorid ließ sich nicht nachweisen.

### Beispiel 2

Es wurde eine Destillation wie in Beispiel 1 durchgeführt, jedoch mit einer Belastung von 0,37 kg/l•h. Der Gehalt an Benzylalkohol in der organischen Phase der Kopffraktion lag unter 4 Gew.-%, der an Benzylchlorid über 96 Gew.-%. Der Benzylchloridgehalt des Sumpfes betrug weniger als 0,01 Gew.-%. Der Zuwachs an Dibenzylether betrug 0,6 Gew.-%, bezogen auf Benzylalkohol.

### Beispiel 3

Beispiel 2 wurde mit einer Belastung von 0,76 kg/l•h wiederholt. Die Kopffraktion setzte sich nach der Gleichgewichtseinstellung in ihrer organischen Phase aus mehr als 96 Gew.-% Benzylchlorid und weniger als 4 Gew.-% Benzylalkohol zuammen; daneben wurde verdünnte Salzsäure durch Absitzenlassen erhalten. Aus dem Sumpf wurde nach Destillation eine Fraktion mit mehr als 99 Gew.-% Benzylalkohol und weniger als 0,05 Gew.-% Benzylchlorid erhalten. Der Zuwachs an Dibenzylether betrug etwa 0,5 Gew.-%, bezogen auf Benzylalkohol.

### Beispiel 4

Beispiel 1 wurde bei einem Druck von 50 mbar und einer Belastung von 0,15 kg/l·h wiederholt. Die Zusammensetzung der organischen Phase des Kopfproduktes betrug durchschnittlich 90 Gew.-% Benzylchlorid und 10 Gew.-% Benzylalkohol. Die Sumpffraktion enthielt weniger als 0,01 Gew.-% Benzylchlorid. Der Zuwachs an Dibenzylether lag bei etwa 1,2 Gew.-%, bezogen auf Benzylalkohol.

### Beispiel 5

Beispiel 1 wurde bei einem Druck von 12 mbar und einer Belastung von 0,11 kg/l·h wiederholt. Die durchschnittliche Zusammensetzung der organischen Phase des Kopfprodukts nach Gleichgewichtseinstellung betrug 85 Gew.-% Benzylchlorid und 15 Gew.-% Benzylalkohol. Die Sumpffraktion enthielt 0,01 Gew.-% Benzylchlorid und weniger als 4,5 Gew.-% Gesamtmenge an Dibenzylether.

## Patentansprüche

1. Verfahren zur Trennung eines Gemisches, das Benzylchlorid, Benzylalkohol, Dibenzylether und wäßrige Salzsäure enthält, dadurch gekennzeichnet, daß ein solches Gemisch einer kontinuierlich betriebenen Destillationskolonne mit Abtriebsteil und Verstärkerteil über eine Seiteneinspeisung zugeführt wird, die Destillationskolonne bei einem Druck von 1 - 950 mbar am Kopf der Kolonne betrieben wird und am Kopf der Destillationskolonne ein Gemisch, das im wesentlichen aus Benzylchlorid und wäßriger Salzsäure besteht, und aus dem Sumpf der Destillationskolonne ein Gemisch, das im wesentlichen aus Benzylalkohol und Dibenzylether besteht, entnommen werden.

2. Verfahren nach Anspruch 1, dadurch gekennzeichnet, daß der Druck am Kopf der Kolonne 10 - 500 mbar, bevorzugt 20 - 300 mbar beträgt.

3. Verfahren nach Anspruch 1, dadurch gekennzeichnet, daß die Temperatur der Kolonne am Einspeisepunkt 25 - 195°C, bevorzugt 55 - 175°C, besonders bevorzugt 65 - 160°C, beträgt.

4. Verfahren nach Anspruch 1, dadurch gekennzeichnet, daß eine Kolonnenbelastung von 0,05 -1,0 kg organische Phase pro Liter Leervolumen der Kolonne pro Stunde, bevorzugt 0,15 - 0,9kg/l•h, besonders bevorzugt 0,25 - 0,8 kg/l•h eingestellt wird.

5. Verfahren nach Anspruch 1, dadurch gekennzeichnet, daß das einzusetzende Gemisch aus 1 - 65 Gew.-% Benzylchlorid, 34 - 98 Gew.-% Benzylalkohol, 0,5 -12 Gew.-% Dibenzylether und 0,5 - 12 Gew.-% wäßriger Salzsäure, bevorzugt aus 9 - 60 Gew.-% Benzylchlorid, 39 - 90 Gew.-% Benzylalkohol, 0,5 - 10 Gew.-% Dibenzylether und 0,5 - 10 Gew.-% wäßriger Salzsäure, besonders bevorzugt 24 - 55 Gew.-% Benzylchlorid, 44 - 75 Gew.-% Benzylalkohol, 0,5 - 8 Gew.-% Dibenzylether und 0,5 - 8 Gew.-% wäßriger Salzsäure besteht, wobei die Angaben auf das Gesamtgewicht der vier Bestandteile bezogen sind.

## Claims

1. Process for the separation of a mixture which contains benzyl chloride, benzyl alcohol, dibenzyl ether and aqueous hydrochloric acid, characterized in that a mixture of this type is fed via a side-feed to a continuously operated distillation column having a stripping section and enrichment section, the distillation column is operated at a pressure of 1-950 mbar at the top of the column, and at the top of the distillation column a mixture which essentially comprises benzyl chloride and aqueous hydrochloric acid is taken off and from the bottom of the distillation column a mixture which essentially comprises benzyl alcohol and dibenzyl ether is taken off.

2. Process according to Claim 1, characterized in that the pressure at the top of the column is 10-500 mbar, preferably 20-300 mbar.

3. Process according to Claim 1, characterized in that the temperature of the column at the feed-in point is 25-195°C, preferably 55-175°C, particularly preferably 65-160°C.

4. Process according to Claim 1, characterized in that a column loading of 0.05-1.0 kg of organic phase per litre of void volume of the column per hour is set, preferably 0.15-0.9 kg/l·h, particularly preferably 0.25-0.8 kg/l·h.

5. Process according to Claim 1, characterised in that the mixture to be used comprises 1-65% by weight of benzyl chloride, 34-98% by weight of benzyl alcohol, 0.5-12% by weight of dibenzyl ether and 0.5-12% by weight of aqueous hydrochloric acid, preferably 9-60% by weight of benzyl chloride, 39-90% by weight of benzyl alcohol, 0.5-10% by weight of dibenzyl ether and 0.5-10% by weight of aqueous hydrochloric acid, particularly preferably 24-55% by weight of benzyl chloride, 44-75% by weight of benzyl alcohol, 0.5-8% by weight of dibenzyl ether and 0.5-8% by weight of aqueous hydrochloric acid, the figures being based on the total weight of the four constituents.

## Revendications

1. Procédé de séparation d'un mélange qui contient du chlorure de benzyle, de l'alcool benzylique, du dibenzyléther et de l'acide chlorhydrique aqueux, caractérisé en ce que l'on envoie un tel mélange, par une alimentation latérale, à une colonne de distillation comportant une partie rectification et une partie concentration, que l'on fait fonctionner en continu, on fait fonctionner la colonne de distillation à une pression de 1-950 mbar à la tête de la colonne et on prélève à la tête de la colonne de distillation un mélange qui consiste essentiellement en chlorure de benzyle et en acide chlorhydrique aqueux, et du pied de la colonne de distillation, un mélange qui consiste essentiellement en alcool benzylique et en dibenzyléther.

2. Procédé selon la revendication 1, caractérisé en ce que la pression à la tête de la colonne est de 10-500 mbar, de préférence de 20-300 mbar.

3. Procédé selon la revendication 1, caractérisé en ce que la température de la colonne au point d'alimentation est de 25-195°C, de préférence de 55-175°C, de manière particulièrement préférée de 65-160°C.

4. Procédé selon la revendication 1, caractérisé en ce que l'on établit une charge de la colonne de 0,05-1,0 kg de phase organique par litre de volume vide de la colonne par heure, de préférence de 0,15-0,9 kg/l.h, de manière particulièrement préférée de 0,25-0,8 kg/l.h.

5. Procédé selon la revendication 1, caractérisé en ce que le mélange à utiliser consiste en 1-65 % en masse de chlorure de benzyle, 34-98 % en masse d'alcool benzylique, 0,5-12 % en masse de dibenzyléther et 0,5-12 % en masse d'acide chlorhydrique aqueux, de préférence en 9-60 % en masse de chlorure de benzyle, 39-90 % en masse d'alcool benzylique, 0,5-10 % en masse de dibenzyléther et 0,5-10 % en masse d'acide chlorhydrique aqueux, de manière particulièrement préférée en 24-55 % en masse de chlorure de benzyle, 44-75 % en masse d'alcool benzylique, 0,5-8 % en masse de dibenzyléther et 0,5-8 % en masse d'acide chlorhydrique aqueux, les données étant rapportées à la masse totale des quatre constituants.
